# EUROPÄISCHE PATENTSCHRIFT

(11) **EP 1 013 307 B1**
(45) Veröffentlichungstag und Bekanntmachung des Hinweises auf die Patenterteilung: **06.03.2002**
(21) Anmeldenummer: 99250410.0
(22) Anmeldetag: 20.11.1999
(51) Int. Cl.: A61N 1/368

(54) **Selbstkalibrierender ratenadaptiver Herzschrittmacher**
Self-calibrating rate adaptive pacemaker
Stimulateur cardiaque à fréquence adaptable et à autocalibration

(30) Priorität: 15.12.1998 DE 19859653
(43) Veröffentlichungstag der Anmeldung: 28.06.2000
(73) Patentinhaber: BIOTRONIK Mess- und Therapiegeräte GmbH & Co Ingenieurbüro Berlin, 12359 Berlin (DE)
(72) Erfinder: Bolz, Armin, 76356 Weingarten (DE)
(74) Vertreter: Eisenführ, Speiser & Partner

(56) Entgegenhaltungen:
- EP-A- 0 565 909
- EP-A- 0 804 938
- WO-A-96/35476
- US-A- 4 867 160
- US-A- 5 645 570

## Beschreibung

Die Erfindung betrifft einen selbstkalibrierenden ratenadaptiven Herzschrittmacher.

Ratenadaptive Herzschrittmacher, bei denen die Stimulationsrate in Abhängigkeit von im Körper des Patienten aufgenommenen Signalen eingestellt wird, die den physiologischen Bedarf des Patienten in Bezug auf die Herzaktivität reflektieren, sind seit längerem bekannt und im klinischen Einsatz. Es sind auch verschiedene Vorschläge zur Selbstadaption (Autokalibration) eines solchen ratenadaptiven Herzschrittmachers gemacht worden.

In WO 93/20889 wird eine Zwei-Sensoren-Anordnung mit einer Schaltung zur Erfassung des Minutenvolumens und einem zusätzlichen Aktivitätsfühler vorgeschlagen, bei der die Stimulationsrate in Abhängigkeit von den für die einzelnen Fühler ableitbaren Zielraten bestimmt wird, und in US 5 065 759 eine Zwei-Sensoren-Anordnung, bei der als "physiologisch exakter", aber langsam ansprechender Parameter das QT-Intervall und als schnell ansprechender Parameter die körperliche Aktivität erfaßt und ausgewertet werden.

Aus der EP 0 147 820 ist zudem ein ratenadaptiver Schrittmacher bekannt, bei dem grundsätzlich einer der beiden Sensoren als sogenannter Closed-Loop-Sensor, der Signale aus dem Herz-Kreislauf-Regelkreis erfaßt, zur Ratenadaption herangezogen wird, der weitere Sensor dagegen lediglich eine Kontrollfunktion hat. Nurwenn über die Kontrollfunktion Fehler in der Ratenadaption festgestellt werden, wird der erste zeitweise durch den Kontroll-Sensor ersetzt oder der Verlauf der Sensorkennlinie neu kalibriert.

In der EP 0 498 533 A1 wird bereits ein mit zwei Sensoren arbeitender ratenadaptiver Schrittmacher mit hämodynamisch kontrollierter Einstellung der oberen Grenzrate vorgeschlagen, wobei für diese Funktion verschiedene Sensoren benannt werden, darunter ein Fühler zur Erfassung von Änderungen der rechtsventrikulären Impedanz.

Die Anmelderin hat in der unveröffentlichten deutschen Patentanmeldung P 198 04 843.2 einen selbstkalibrierenden ratenadaptiven Schrittmacher vorgeschlagen, bei dem mittels eines Beschleunigungssensors ein auf intraventrikulär erfaßten Impedanzsignalen beruhender "Closed-Loop"-Ratenadaptionsalgorithmus kalibriert wird.

Im Rahmen des autonomen Systems der Herz-/Kreislauf-Steuerung vom Sympathikus ausgehende ("sympathische") Signale zeigen hauptsächlich den Bedarf an Herzminutenvolumen an und finden im nervalen Gleichgewicht des gesunden Herz-Kreislauf-Systems ihren Gegenspieler in vom Vagus ausgehenden ("vagalen" oder "parasympathischen") Signalen, die das Erreichen von Obergrenzen für die Leistungsfähigkeit des Herzminutenvolumens anzeigen. Im Gegensatz zu sympathischen Signalen wirken sich vagale also hemmend aus.

Bei den bekannten ratenadaptiven Schrittmachern wird im wesentlichen die Änderung der Inotropie gemessen und als Maß für das erforderliche Herzminutenvolumen und damit die optimale Stimulationsrate, genutzt. Dabei wird eine lineare Beziehung zwischen Inotropie und Herzrate angenommen, d.h. ein Anstieg der Inotropie wird sofort mit einem proportionalen Anstieg der Stimulationsrate beantwortet. Durch die Messung der Inotropie über die ventrikuläre Kontraktionsdynamik (mittels unipolarer Impedanzmessung) wird vorwiegend der sympathische Anteil der autonomen Regelung erfaßt. Vagale Anteile und ihre (in der Regel hemmende) Wirkung auf die Herzrate werden praktisch nicht erfaßtund berücksichtigt. Dieser "rein sympathische" Schrittmacher funktioniert folglich - zumindest in Bezug auf die Herzrate - in Analogie zu einem Patienten mit geringer Barorezeptorreflexsensitivität: Der vagale Tonus ist künstlich auf Null reduziert bzw. auf eine konstanten Wert gesetzt, und der Sympathikotonus wirkt allein steuernd. Dies hat zwei Nachteile zur Folge: Zum einen sind damit schnelle Herzratenanpassungen, wie sie mit vagaler Beteiligung beim funktionierenden Herzen möglich sind, vom Schrittmacher nicht nachvollziehbar. Zum anderen kommt auch die die Herzratendynamik steuernde Funktion des Vagus nicht zur Wirkung. Das bedeutet, daß Langzeiteffekte (bspw. allg. körperliche Ermüdung - der sogenannte "burn-out" -, Vorboten einer beginnenden Herz-insuffizienz etc.) im wesentlichen unberücksichtigt bleiben.

Dadurch wird aber der eigentliche Vorteil der autonomen Kontrolle, nämlich die Schonung der inotropen Reserven und damit der Schutz vor primären Kardiomyopathien bzw. Arrhythmien, nicht in optimaler Weise ausgenutzt. Infolge der Vernachlässigung des vagalen Beitrages ist die berechnete Stimulationsrate in ihrer absoluten Höhe nicht physiologisch korrekt, wenn auch Abfalls- und Anstiegszeiten richtig sein mögen. Dadurch wird das Myokard u.U. überlastet oder nicht ausreichend belastet.

Es ist Aufgabe der Erfindung, einen aus physiologischer Sicht optimiert und zuverlässig selbstkalibrierend arbeitenden und unproblematisch zu realisierenden Herzschrittmacher der gattungsgemäßen Art anzugeben.

Diese Aufgabe wird durch einen Herzschrittmacher mit den in Anspruch 1 angegebenen Merkmalen gelöst.

Die Erfindung beruht auf dem grundlegenden Gedanken, einen mit angemessenem vagalem Steuerungsbeitrag arbeitenden Schrittmacher anzugeben, der Signale von zwei Closed-Loop-Sensoren zur Ratensteuerung nutzt, nämlich einem nachfolgend kurz als "sympathischer" Sensor und einem als "vagaler" Sensor bezeichneten. Der Einfluß des Vagus schlägt sich hauptsächlich in der elektrischen und mechanischen Aktivität der Atria, also speziell etwa der atrialen evozierten Reizantwort {AER - unipolar zu messen), dem atrialen monophasischen Aktionspotential {MAP-bipolar zu messen}, der atrialen Refraktärzeit, der intraatrialen Impedanz und auch der AV-Überleitungszeit nieder, der Einfluß des Sympathikus hauptsächlich in der Aktivität des Ventrikels, speziell der ventrikulär evozierten Reizantwort {VER), dem ventrikulären monophasischen Aktionspotential {MAP), derventrikulären Refraktärzeit, der intraventrikulären Impedanz und dem QT-Intervall. Ein Sensor für vom Sympathikus dominierte Signale wird als "sympathischer" Sensor bezeichnet, und ein Sensor für vom Vagus dominierte Signale als "vagaler" Sensor. Sympathische Signale reagieren auf Veranderungen im Herz-Kreislaufsystem langsamer (mit einer Zeitkonstanten von etwa 10 Herzzyklen) als vagale Signale (mit einer Zeitkonstanten von etwa einem Herzzyklus), und entsprechend unterschiedlich ist auch das Sensor-Zeitverhalten.

Weiter schließt die Erfindung den Gedanken ein, die aktuelle Steuerung der Stimulationsfrequenz bei der "Closed-Loop"-Stimulation (CLS) grundsätzlich anhand der Signale des "sympathischen" Sensors auszuführen. Entweder werden relative Änderungen des Sensorsignals nach Maßgabe der Ansprechverstärkung oder des Response-Faktors (der ein Maß für die Sensor-Dynamik dastellt) in dazu direkt proportionale Stimulationsfrequenzen umgesetzt, oder absolute Sensor-Werte werden über eine Kennlinie in Ratensteuersignale umgesetzt.

Der vagale Sensor wird zur Kalibration des sympathischen Sensors oder, genauer gesagt, der Sensor-Dynamik (Response-Faktor oder Steigung der Sensorkennlinie) und des bei der aktuellen Steuerung überstreichbaren Ratenbereiches genutzt. Werden beispielsweise durch den Ratenadaptionsalgorithmus anhand des sympathischen Sensors dynamisch überhöhte oder grundsätzlich über der Leistungsfähigkeit des HerzKreislaufsystems, d.h. außerhalb des hämodynamisch begründeten Ratenbereiches liegende Stimulationsraten ermittelt, so wird durch den vagalen Sensor die hemmende Einflußnahme des vagalen Nervensystems zur Geltung gebracht. Ist andererseits der Vagotonus gering, so werden Response-Faktor und obere Grenzrate erhöht und hierdurch der Symathikotonus gesenkt, woraufhin ein Anstieg des Vagotonus zu erwarten ist.

Weiterhin ist im Rahmen der Erfindung (ungeachtet der oben erwähnten Annahme der grundsätzlichen Dominanz sympatischer oder vagaler Anteile in bestimmten Arten von Herzsignalen) die Trennung sympathischer und vagaler Signalanteile in den verfügbaren, komplex determinierten Signalen von besonderer Bedeutung. Eine meßtechnisch günstige und dem Verhalten des gesunden Herz-Kreislaufsystems sehr nahe kommende Möglichkeit zur Separation der vagalen und sympathischen Signale basiert auf einer Modulation der beiden Toni und der Analyse der Antworten eines geeignet ausgewählten Effektors unter Ausnutzung der unterschiedlichen Zeitkonstanten. Es wird also die Stimulationsrate um die adaptive Rate herum moduliert. Eine schnelle Modulation der Stimulationsfrequenz (speziell auf Schlagzu-Schlag-Basis) kann eine ebenfalls schnelle Variation der Signale des vagalen Sensors hervorrufen, die wiederum im optimalen Leistungsbereich des Herzens ein bestimmtes Ausmaß annehmen muß. Dagegen zieht eine langsame (sich etwa über 10 Herzzyklen erstreckende) Modulation der Stimulationsfrequenz eine Variation der sympathischen Signale nach sich.

In einer bevorzugten Ausführung nutzt der Schrittmacher als sympathisches Signal zur Ratenadaption die intraventrikuläre Impedanz und als vagales Signal zur Kalibration die natürliche AV-Überleitungszeit.

Durch Erfassung und geeignete Auswertung der intraventrikulären Impedanz wird im Grunde die Inotropie (Schlagstärke oder -kraft des Herzmuskels) gemessen und daraus die Stimulationsrate errechnet. Hierbei können sich beispielsweise bei ansteigender Belastung und zu großem Response-Faktor überhöhte Stimulationsraten ergeben. Darauf reagiert die AV-Überleitungszeit - die sich bei physiologisch angemessen ansteigenden Ratenwerten grundsätzlich verkürzt - mit einer unnatürlichen Verlängerung. Umgekehrt reagiert die AV-Uberleitungszeit auf einen in Bezug zum hämodynamischen Bedarf zu geringen Anstieg der Stimulationsfrequenz mit einer unnatürlichen Verkürzung. Wird das erwähnte unnatürliche Verhalten der AV-Uberleitungszeit festgestellt, kann der Response-Faktor korrigiert werden.

Aufgrund dieser Kalibration kann zugleich die obere Grenze MCLR ("maximum closed-loop rate") für den Sensor-Ratenbe-reich dynamisch festgelegt werden, da bei Überschreitung der maximalen physiologischen Stimulationsrate ebenfalls die beschriebene unnatürliche Verlängerung der AV-Uberleitungszeit festzustellen ist.

Die Kalibration sowohl der Sensor-Dynamik als auch des Sensor-Ratenbereichs läßt sich aufgrund einer Modulation der Stimulationsfrequenz vornehmen. Bei schneller Modulation wird sich die Variation der AV-Zeit bei überhöhten Stimulationsraten im Vergleich zu ihrer Variation bei physiologischen Stimulationsraten zu längeren AV-Zeiten hin verschieben; dies gilt sowohl für momentan überhöhte als auch für über der Leistungsfähigkeit des Herzens liegende Stimulationsraten.

Die Auswertung der Impedanzmessungen erfolgt beispielsweise gemäß dem an sich bekannten ResQ-bzw. Steigungsverfahren (SCHALDACH, Max: Electrotherapy of the Heart, 1. Aufl., Springer-Verlag, S. 114 ff.) in einem breiten Bereich, der die üblicherweise für einzelne Patienten eingestellten ROI-Bereiche einschließt. Auch die Abtastzeitpunkt-Paare sind so vorprogrammiert, daß sie mit Sicherheit das für den betreffenden Patienten "optimale" Paar enthalten. Ihre Festlegung bedarf jedoch keiner patientenindividuellen Programmierung nach der Implantation, sondern sie werden bevorzugt bei der Herstellung des Schrittmachers in einem Festwertspeicher gespeichert. Grundsätzlich kann die Impedanzerfassung und -auswertung an einem spontanen ventrikulären Signal erfolgen, aufgrund der hierbei vielfach ungünstigen und schwankenden Signalform wird die Erfassung an einem evozierten Signal (VER) aber in der Praxis bevorzugt. Bezugspunkt für die Wahl des Abtastzeitpunkt-Paares ist dann der ventrikuläre Stimulus.

Mit geringerem Kalibrationsaufwand kommt ein Verfahren aus, bei dem anhand mehrerer Stützstellen ein Wert für das Integral der Impedanzkurve ermittelt und die Ratenadaption aufgrund von Änderungen dieses Integralwertes ausgeführt wird; vgl. dazu DE 196 09 382 A1.

Die die Abhängigkeit der Stimulationsrate von der Impedanzgröße bestimmende Kennlinie als erster wesentlicher Betriebsparameter der Ratenbestimmungseinrichtung ist nicht statisch, sondern wird kontinuierlich oder in bestimmten Zeitabständen aufgrund der Erfassung der AV-Überleitungszeit bei modulierter Stimulationsrate optimiert. Auch die Variationsbreite bzw. speziell die MCLR als zweiter wesentlicher Parameter ist bei dieser Ausführung zu Beginn des Betriebs nicht bekannt, sondern wird laufend während des Betriebs optimiert. Zu Beginn des Betriebs wird jeweils ein Schätzwert für den unteren und den oberen Grenzwert der Variationsbreite als Startwert vorgegeben.

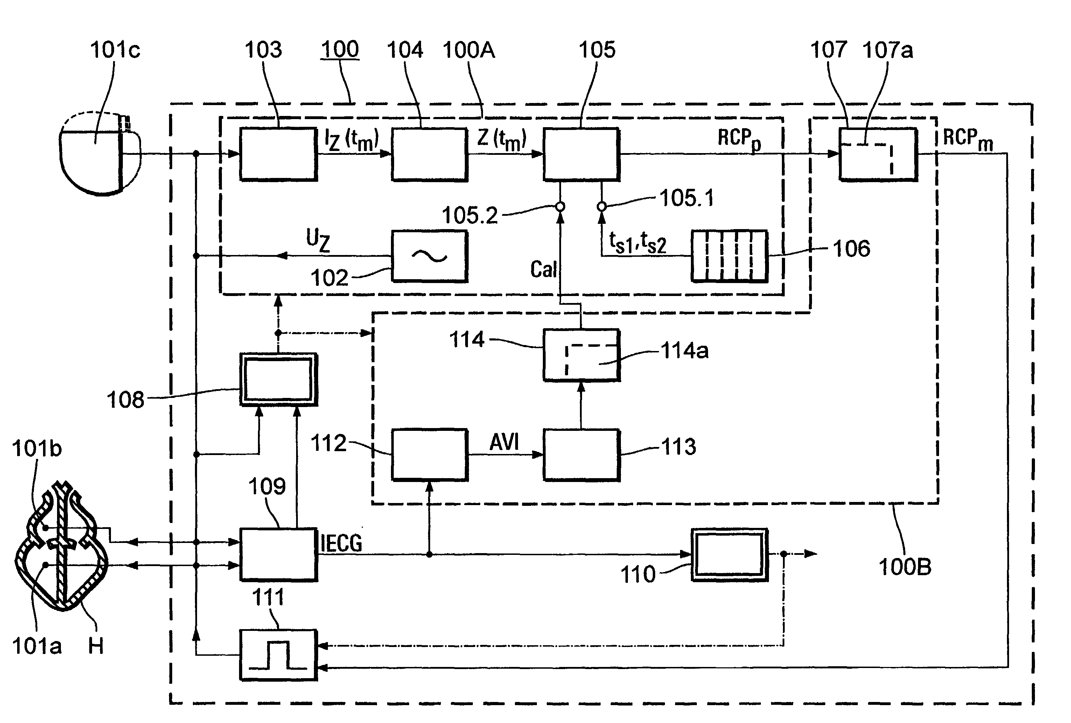

Vorteilhafte Weiterbildungen der Erfindung sind im übrigen in den Unteransprüchen gekennzeichnet bzw. werden nachstehend zusammen mit der Beschreibung einer bevorzugten Ausführung der Erfindung anhand der Figur näher dargestellt. Diese zeigtalsAusführungsbeispiel derErfindung einen ratenadaptiven Herzschrittmacher 100 als fragmentarisches Funktions-Blockschaltbild, in dem nur die für die Erläuterung der Erfindung wichtigen Komponenten dargestellt sind. Der übrige Aufbau entspricht dem bekannter Herzschrittmacher.

An den Herzschrittmacher 100 sind eine unipolare Schrittmacher- und Meßelektrode 101 a im rechten Ventrikel des Herzens H zur Herzsignalerfassung, zur Messung der rechts-ventrikulären intrakardialen Impedanz Z und als Reizelektrode und eine atriale Abfühl- und Stimulationselektrode 101 b angeschlossen. Als Gegenelektrode dient eine Schrittmachergehäuseelektrode 101c.

Die Impedanzmessung erfolgt in an sich bekannter Weise, indem die Elektrode 101 a über einen Meßspannungsgenerator 102 mit einer Meßspannung U_{Z} beaufschlagt und über eine Strommeßschaltung 103 zu m äquidistant vorprogrammierten Zeitpunkten tₘ der Strom I_{Z}(tₘ) zwischen der Meßelektrode 101a und der Gegenelektrode 101b gemessen wird. Die den zeitlichen Verlauf der Impedanz kennzeichnenden Werte Z(tₘ) ergeben sich am Ausgang einer der Strommeßschaltung 103 nachgeschalteten Impedanzberechnungsstufe 104 als Quotienten aus der (fest voreingestellten) Meßspannung und den gemessenen Stromwerten. In einer RCP-Berechnungsstufe 105, die über einen ersten Steuereingang 105.1 mit einem Abtastzeitspeicher 106 verbunden ist und über einen zweiten Steuereingang 105.2 ein Responsefaktor-Steuersignal empfängt, wird für jeden erfaßten Impedanz-Zeitverlauf Z(tₘ) ein primärer Ratensteuerparameterwert RCPₚ berechnet.

In einer der RCP-Berechnungsstufe 105 nachgeschalteten Modulatorstufe 107 wird den primären RCP-Werten ein in einem Musterspeicher 107a gespeichertes vorbestimmtes Modulationsmuster mit relativ kurzer Periodendauer von einigen Herzzyklen aufgeprägt, und am Ausgang der Modulatorstufe 107 steht zu jedem Stimulationszeitpunkt ein gemäß dem Modulationsmuster variierter ("modulierter") RCP-Wert RCPₘ zur Verfügung.

Die oben erläuterten Komponenten 102 bis 106 bilden eine Impedanzverarbeitungseinrichtung 100A des Schrittmachers 100, mit der die Sympathikus-Komponente bei der CLS zur Geltung gebracht wird.

Eine Ratenadaptions-Zeitsteuerung 108 steuert sowohl die oben skizzierte Impedanzerfassung und -auswertung in der Stufe 100A und die "schnelle" Modulation des Ratensteuersignals in der Stufe 107 als auch die nachfolgend beschriebene Erfassung und Auswertung der AV-Überleitungszeit (AVI). Die Impedanzmessung und AVI-Erfassung werden durch die Ablaufsteuerung in Synchronität zur Herzaktivität bzw. Schrittmacherstimulation ausgelöst. Der Schrittmacher umfaßt hierzu in üblicher Weise eine IECG-Eingangsstufe 109 (die in praxi durch separate Atrium- und Ventrikel-Eingangsstufen gebildet sein kann), eine Schrittmacherbetriebssteuerung 110 und eine Ausgangsstufe 111 (die analog zur Eingangsstufe separate Atrium- und Ventrikel-Stufen umfassen kann) sowie weitere an sich bekannte Komponenten. Die allgemeine Funktion dieser Komponenten wird hier als bekannt vorausgesetzt.

Für die hier beschriebene Kalibration ist darauf hinzuweisen, daß der Schrittmacher entweder im Vorhofstimulations-Modus arbeitet - was nachfolgend vorausgesetzt wird - und die Impedanz dann am natürlichen ventrikulären Ereignis auszuwerten ist oder während der Kalibration Betriebsartumschaltungen vorzusehen sind, um einerseits die Auswertung der Impedanz an der VER vornehmen zu können und andererseits das natürliche AV-Intervall verfügbar zu haben.

Der IECG-Eingangsstufe 109 ist eine AVI-Erfassungsstufe 112 nachgeschaltet, und deren Ausgang ist zu einer AVI-Trendermittlungsstufe 113 geführt, die einen AVI-Speicher 113a für eine vorbestimmte Anzahl von Vergangenheits-AVI-Werten (mindestens den vorletzten Wert) umfaßt und in der bestimmt wird, ob (und ggfs. in welcher Ausprägung) ein Trend zur Verlängerung oder Verkürzung des AVI gegenüber dem vorangegangenen Herzzyklus oder den vorangegangenen Zyklen besteht. Mit den Ausgängen der AVI-Trendermittlungs-stufe 113 und der Modulatorstufe 107 ist ein Kalibrationssignalgenerator 114 verbunden, in dem das Ergebnis der AVI-Trendermittlung mit dem zugrundeliegenden Modulationsvorgang korreliert und anhand einer in einem internen Speicher 114a gespeicherten Entscheidungsmatrix ein Korrektur- oder Verifikationssignal als Responsefaktor-Steuersignal Cal bestimmt und an den Steuereingang 105.2 der RCP-Berechnungsstufe 105 übermittelt wird. In der als Beispiel skizzierten Ausführung wird ein ausgegebenes Steuersignal unmittelbar für den nächsten Herzzyklus kalibrationswirksam. Es ist aber auch möglich, eine Korrektur (oder Verifikation) des Response-faktors jeweils erst nach Abschluß eines Modulationszyklus oder auch mehrerer Zyklen vorzunehmen und zu diesem Zweck (nicht gezeigte) Mittel zur Zwischenspeicherung oder zur zeitlichen Mittelung des Respon-sefaktor-Steuersignals vorzusehen.

Die Entscheidungsmatrix ist im einfachsten Falle eine 2x2-Matrix, in der die Möglichkeiten aufwärts oder abwärts modulierter Rate (RCPₘ > RCPₚ bzw. RCPₘ < RCPₚ) mit der Folge verlängerter oder verkürzter AV-Überleitungszeit (AVI↑ bzw. AVI↓) über vier Steuersignal-Felder verknüpft sind, die eines der Responsefaktor-Steuersignale "Erhöhen" (RF↑), "Verringern" (RF↓) oder "Beibehalten" (RF→) enthalten:

| AVI \| Mod. | RCPₘ > RCPₚ | RCPₘ < RCPₚ |
|---|---|---|
| AVI↑ | RF↓ | RF→ |
| AVI↓ | RF→ | RF↑ |

Diese Matrix trägt dem Umstand Rechnung, daß die AV-Überleitungszeit sich bei physiologisch angemessen ansteigender Rate verkürzt bzw. bei sinkender Rate verlängert, während "unphysiologische" Ratenänderungen gerade das entgegengesetzte Verhalten des AVI provozieren. Durch Berücksichtigung des Falles gleichbleibender AV-Zeit und Differenzierung nach dem Betrag der jeweiligen Änderungen in einer entsprechend vergrößerten Matrix läßt sich ein verfeinerter und noch schneller ansprechender Kalibrationsalgorithmus ableiten. Es ist darauf hinzuweisen, daß mittels der oben beschriebenen oder einer ähnlichen Matrix neben dem Responsefaktor im Grunde auch die MCLR kalibriert werden kann: Bei hoher Belastung des Patienten wird nämlich - wie in jedem anderen Belastungszustand - die eine unphysiologische Ratenerhöhung anzeigende Verlängerung des AVI eine Verringerung des Responsefaktors auslösen, und zwar solange, bis keine nennenswerte Steigerung der Stimulationsrate mehr eintritt, was eine effektive obere Ratenbegrenzung ergibt.

Die Baugruppen 107 und 112 bis 114 bilden die Kalibrationsstufe 100B des Schrittmachers, mit der die Vagus-Komponente bzw. der parasympathische Einfluß bei der CLS zur Geltung gebracht wird.

Die Signalverbindung der Schrittmachersteuerung 110 zu den intrakardialen Elektroden 101a, 101b, mit der die Herzaktionen bzw. intrakardialen EKGs erfaßt werden, ermöglicht übrigens eine Unterscheidung zwischen spontanen und evozierten Herzaktionen und damit die Berücksichtigung des Ereignistyps bei der impedanzbasierten Ratenberechnung. Insbesondere kann der errechneten Stimulationsrate durch die Schrittmachersteuerung bei jedem Ereignistypwechsel ein Wert aus einer Mehrzahl intern gespeicherter Ratenoffsetwerte hinzugefügt werden, dessen Betrag in Abhängigkeit vom vorhergehenden und vom aktuellen RCP-Wert so gewählt ist, daß der Ratensprung einen vorbestimmten Betrag nicht überschreitet, und der bei den dann nachfolgenden Herzereignissen stufenweise bis auf Null zurückgeführt wird. Die konkreten schaltungstechnischen Mittel zur Realisierung dieser Zusatzfunktion stehen dem Fachmann aus bekannten Anordnungen zur Glättung von Sprungfunktionen von Steuergrößen, speziell zur Ratenverlaufs-Glättung bei Schrittmachern, zur Verfügung.

Die Erfindung beschränkt sich in ihrer Ausführung nicht auf die vorstehend angegebenen bevorzugten Ausführungsbeispiele. Vielmehr ist eine Anzahl von Varianten denkbar, welche von der beanspruchten Lösung auch in anders gearteter Ausführung Gebrauch machen.

So können anstelle der oben genannten Sensoren auch andere sympathisch bzw. vagal dominierte Sensoren eingesetzt werden. Zur zusätzlichen Berücksichtigung des Orthostaseeffekts kann zusätzlich ein Positionsfühler vorgesehen sein, dessen Signale über einen geeigneten Algorithmus die Verarbeitung des Impedanzsignals beeinflussen.

In einer weiteren modifizierten Ausführung ist neben den beiden Closed-Loop-Sensoren zusätzlich ein Aktivitätsfühler oder ein anderer "Nicht-Closed-Loop"-Sensor vorgesehen, der die Anwendungsbreite und Zuverlässigkeit des Kalibrationsalgorithmus weiter erhöhen und ggfs. die Ratensteuerung übernehmen kann, falls (beispielsweise aufgrund von Störungen oder eines veränderten Krankheitsbildes) über längere Zeiträume keine Autokalibration der CLS gemäß dem programmierten Algorithmus möglich sein sollte.

## Patentansprüche

1. Selbstkalibrierender ratenadaptiver Herzschrittmacher (100), mit einer ersten Meß- und Verarbeitungseinrichtung (100A) zur Erfassung eines ersten, vorherrschend sympathisch beeinflußten physiologischen Parameters (Z(tₘ)) und zur Gewinnung eines Ratensteuerparameters (RCPₚ), die einen Steuereingang (105.2) zur Steuerung der funktionalen Abhängigkeit des Ratensteuerparameters vom ersten physiologischen Parameter, insbesondere eines Responsefaktors und/oder einer oberen Grenzrate, aufweist, und einer Stimulatoreinheit (110, 111) zur Erzeugung und Ausgabe von Stimulationsimpulsen mit einer durch den ratensteuerparameter bestimmten Stimulationsrate, mit einer zweiten Meß- und Verarbeitungseinrichtung (100B) zur Erfassung und Auswertung eines zweiten, vorherrschend vagal beeinflußten physiologischen Parameters (AVI) und zur Ausgabe eines vom Auswertungsergebnis abhängigen Kalibrationssignals (Cal) an den Steuereingang (105.2) der ersten Meß- und Verarbeitungseinrichtung (100A).

2. Herzschrittmacher nach Anspruch 1, **dadurch gekennzeichnet, daß** die erste Meß- und Verarbeitungseinrichtung (100A) zur Messung und Verarbeitung eines die ventrikuläre Herzaktivität kennzeichnenden Parameters und die zweite Meß- und Verarbeitungseinrichtung (100B) zur Messung und Verarbeitung eines die atriale Herzaktivität oder die zeitliche Korrelation zwischen atrialer und ventrikulärer Aktivität kennzeichnenden Parameters ausgebildet ist.

3. Herzschrittmacher nach Anspruch 1 oder 2, **gekennzeichnet durch** Mittel zur Abtrennung von vagal beeinflußten Anteilen im Ausgangssignal (RCPₚ) der ersten Meß- und Verarbeitungseinrichtung (100A) und/oder von sympathisch beeinflußten Anteilen im Ausgangssignal (Cal) der zweiten Meß- und Verarbeitungseinrichtung (100B).

4. Herzschrittmacher nach einem der vorangehenden Ansprüche, **dadurch gekennzeichnet, daß** eine, insbesondere den Mitteln zur Abtrennung zugehörige, Einrichtung (107) zur Erzeugung eines modulierten Ratensteuersignals (RCPₘ) mit einer der typischen Zeitkonstanten sympathisch oder vagal gesteuerter Aktivität entsprechenden Modulationsrate und eine Einrichtung (113) zur Auswertung des Einflusses der Modulation auf das jeweilige Ausgangssignal (Cal) vorgesehen ist.

5. Herzschrittmacher nach Anspruch 4, **dadurch gekennzeichnet, daß** die Einrichtung (107) zur Erzeugung eines modulierten Ratensteuersignals (RCPₘ) zur Auswertung des Einflusses auf das Ausgangssignal (Cal) der zweiten Meß- und Verarbeitungseinrichtung (100B) eine Modulation mit einer annähernd einem Herzzyklus bzw. Stimulationsintervall entsprechenden Zeitkonstanten ausführt.

6. Herzschrittmacher nach einem der Ansprüche 2 bis 5, **dadurch gekennzeichnet, daß** die erste Meß- und Verarbeitungseinrichtung als Impedanzmeß- und -verarbeitungsein-richtung (100A) zur Erfassung der rechtsventrikulären Impedanz und zur Bestimmung des Anstieges oder des Integrals der Impedanz-Zeit-Funktion in einem vorbestimmten Abtastzeitintervall ausgebildet ist.

7. Herzschrittmacher nach einem der Ansprüche 2 bis 6, **dadurch gekennzeichnet, daß** die zweite Meß- und Verarbeitungseinrichtung (100B) zur Erfassung und Auswertung der natürlichen AV-Überleitungszeit (AVI) ausgebildet ist.

8. Herzschrittmacher nach einem der vorhergehenden Ansprüche, **gekennzeichnet durch** einen zusätzlichen, nicht auf ein Signal aus dem Herz/Kreislauf-Regelkreis ansprechenden Körperfühler, insbesondere einen Aktivitäts- oder Orthostasesensor.

## Claims

1. A self-calibrating rate-adaptive cardiac pacemaker (100), comprising a first measuring and processing device (100A) for detecting a first, predominantly sympathetically influenced physiological parameter (Z(tₘ)) and for obtaining a rate control parameter (RCPₚ), which has a control input (105.2) for controlling the functional dependency of the rate control parameter on the first physiological parameter, in particular a response factor and/or an upper limit rate, and a stimulator unit (110, 111) for producing and outputting stimulation pulses at a stimulation rate which is determined by the rate control parameter, and comprising a second measuring and processing device (100B) for detecting and evaluating a second, predominantly vagally influenced physiological parameter (AVI) and for. outputting. a calibration signal (Cal) which is dependent on the evaluation result, to the control input (105.2) of the first measuring and processing device (100A).

2. A cardiac pacemaker according to claim 1 **characterised in that** the first measuring and processing device (100A) is adapted for measuring and processing a parameter which characterises ventricular cardiac activity and the second measuring and processing device (100B) is adapted for measuring and processing a parameter which characterises atrial cardiac activity or the time correlation between atrial and ventricular activity.

3. A cardiac pacemaker according to claim 1 or claim 2 **characterised by** means for separating vagally influenced components in the output signal (RCPₚ) of the first measuring and processing device (100) and/or sympathetically influenced components in the output signal (Cal) of the second measuring and processing device (100B).

4. A cardiac pacemaker according to one of the preceding claims **characterised in that** there is provided a device (107), which in particular is associated with said separation means, for producing a modulated rate control signal (RCPₘ) with a modulation rate corresponding to the typical time constant of sympathetically or vagally controlled activity, and a device (113) for evaluation of the influence of modulation on the respective output signal (Cal).

5. A cardiac pacemaker according to claim 4 **characterised in that** the device (107) for producing a modulated rate control signal (RCPₘ) for evaluating the influence on the output signal (Cal) of the second measuring and processing device (100B) implements modulation with a time constant which approximately corresponds to a cardiac cycle or stimulation interval.

6. A cardiac pacemaker according to one of claims 2 to 5 **characterised in that** the first measuring and processing device is in the form of an impedance measuring and processing device (100A) for detecting the right-ventricle impedance and for determining the rise or the integral of the impedance-time function in a predetermined sensing time interval.

7. A cardiac pacemaker according to one of claims 2 to 6 **characterised in that** the second measuring and processing device (100B) is adapted for detecting and evaluating the natural AV-conduction time (AVI).

8. A cardiac pacemaker according to one of the preceding claims **characterised by** an additional body sensor which is not responsive to a signal from the heart-circulation regulating circuit, in particular an activity or orthostasis sensor.

## Revendications

1. Simulateur cardiaque à calibrage automatique et à cadence adaptative (100), comportant un premier dispositif de mesure et de traitement (100A) pour détecter un premier paramètre physiologique (Z(tₘ)) influencé de façon prépondérante au niveau sympathique, et pour obtenir un paramètre de commande de cadence (RCPₚ) qui comporte une entrée de commande (105.2) pour commander la dépendance fonctionnelle du paramètre de commande de cadence vis-à-vis du premier paramètre physiologique, notamment un facteur de réponse et/ou une cadence limite supérieure, et une unité formant simulateur (110, 111) pour produire et délivrer des impulsions de stimulation avec une cadence de stimulation déterminée par le paramètre de commande de cadence, et comportant un second dispositif de mesure et de traitement (100B) pour détecter et évaluer un second paramètre physiologique (AVI), influencé de façon prépondérante au niveau vagal, et servant à délivrer un signal de calibrage (Cal), qui dépend du résultat de l'évaluation, à l'entrée de commande (105.2) de l'unité de mesure et de traitement (100A).

2. Stimulateur cardiaque selon la revendication 1, **caractérisé en ce que** le premier dispositif de mesure et de traitement (100A) est conçu pour la mesure et le traitement d'un paramètre caractérisant l'activité cardiaque ventriculaire et que le second dispositif de mesure et de traitement (100B) est conçu pour la mesure et le traitement d'un paramètre caractérisant l'activité cardiaque atriale ou la corrélation dans le temps entre les activités atriale et ventriculaire.

3. Stimulateur cardiaque selon la revendication 1 ou 2, **caractérisé par** des moyens pour séparer des composantes, influencées au niveau vagal, et présentes dans le circuit de sortie (RCPₚ) du premier dispositif de mesure et de traitement (100A) et/ou de composantes, influencées au niveau sympathique et présentes dans le signal de sortie (Cal) du second dispositif de mesure et de traitement (100B).

4. Stimulateur cardiaque selon l'une des revendications précédentes, **caractérisé en ce qu'**il est prévu un dispositif (107), qui est associé notamment aux moyens de séparation et sert à produire un signal de commande de cadence modulé (RCPₘ) avec une cadence de modulation qui correspond à la constante de temps typique d'une activité commandée au niveau sympathique ou vagal, et un dispositif (113) pour évaluer l'influence de la modulation sur le signal de sortie respectif (Cal).

5. Stimulateur cardiaque selon la revendication 4, **caractérisé en ce que** le dispositif (107) servant à produire un signal modulé de commande de cadence (RCPₘ) exécute, pour l'évaluation de l'influence sur le signal de sortie (Cal) du second dispositif de mesure et de traitement (100B), une modulation avec une constante de temps correspondant approximativement à un cycle cardiaque ou à un intervalle de stimulation.

6. Stimulateur cardiaque selon l'une des revendications 2 à 5, **caractérisé en ce que** le premier dispositif de mesure et de traitement est agencé sous la forme d'un dispositif de mesure et de traitement d'impédance (100A) pour détecter l'impédance du ventricule droit et servant à déterminer l'accroissement ou l'intégrale de la fonction de l'impédance dans le temps, dans un intervalle de temps d'échantillonnage prédéterminé.

7. Stimulateur cardiaque selon les revendications 2 à 6, **caractérisé en ce que** le second dispositif de mesure et de traitement (100B) est conçu pour déterminer et évaluer le temps de transition (AV) naturel (AVI).

8. Stimulateur cardiaque selon l'une des revendications précédentes, **caractérisé en ce qu'**un détecteur corporel supplémentaire, qui ne répond pas à un signal provenant du circuit de régulation coeur/ circulation, notamment un capteur d'activité ou d'orthostase.
